# EUROPEAN PATENT SPECIFICATION

(11) **EP 1 504 725 B1**
(45) Date of publication and mention of the grant of the patent: **03.10.2007**
(21) Application number: 04025903.8
(22) Date of filing: 04.04.2001
(51) Int. Cl.: A61B 18/08, A61B 5/00

(54) **Thermography imaging**
Thermographiebildgebung
Imagerie de thermographie

(30) Priority: 04.04.2000 WO PCT/BE00/00031; 17.04.2000 WO PCT/BE00/00038; 21.08.2000 WO PCT/BE00/00096; 08.09.2000 EP 00870196; 16.03.2001 GB 0106614
(43) Date of publication of application: 09.02.2005
(62) Divisional of application: 01929576.5
(73) Proprietor: Thermocore Medical Systems N.V., 9820 Merelbeke, Gent (BE)
(72) Inventor: Diamantopoulos, Leonidas, Guldensporenpark 32 9820 Merelbeke (BE); Van Langenhove, Glenn, Guldensporenpark 32 9820 Merelbeke (BE)
(74) Representative: Finnie, Peter John

(56) References cited:
- EP-A- 0 856 278
- EP-A- 0 943 293
- WO-A-00/13603
- WO-A-92/20290
- US-A- 5 237 996
- US-A- 5 345 938
- US-A- 5 771 895

## Description

### Field of the Invention

The present invention relates to medical devices and methods for measuring and mapping the temperature of vascular tissue. The present invention is particularly related to locating inflamed or unstable artherosclerotic plaque in a blood vessel.

### Background to the Invention

Plaque can develop in a patient's cardiovascular system. The plaque can be quite extensive and occlude a substantial length of the vessel. Additionally, the plaque may be inflamed and unstable, such plaque being subject to rupture, erosion or ulceration which can cause the patient to experience a myocardial infarction, thrombosis or other traumatic and unwanted effects. Furthermore, relative blood viscosity rises and aggregation of platelets increases with temperature increases (Dintefass L. Rheology of Blood in Diagnostic and Preventive Medicine. London, UK: Butterworths; 1976;66-74). Previous ex vivo studies have shown that there is indeed thermal heterogeneity in human carotid atherosclerotic plaques (Casscells W, Hathorn B, David M, Krabach T, Vaughn WK, McAllister HA, Bearman G, Willerson JT. Thermal detection of cellular infiltrates in living atherosclerotic plaques: possible implications for plaque rupture and thrombosis. Lancet. 1996;347:1447-1449).

Presently, a number of procedures are available for visualising the morphology of a blood vessel, thus locating areas of atherosclerosis.

Angiography is used to detect abnormalities or occlusions in the blood vessels throughout the circulatory system and in some organs. The procedure is commonly used to identify atherosclerosis; to diagnose heart disease; to evaluate kidney function and detect kidney cysts, or tumors; to detect an aneurysm, tumor, blood clot, or arteriovenous malformations in the brain; and to diagnose problems with the retina of the eye.

Angiography requires the injection of a contrast medium that makes the blood vessels visible to x-ray. The dye is injected through a procedure known as arterial puncture. The puncture is usually made in the groin area, armpit, inside elbow, or neck.

In particular, for cardiac angiography, the puncture is generally made in the femoral artery. A needle containing a stylet is inserted into the artery. When the artery has been punctured with the needle, the stylet is removed and replaced by an intravascular sheath, through which a guiding catheter is placed inside the femoral artery. Once the guide catheter has been placed close to the artery of interest, for example, the coronary arteries, a guide wire may be inserted into the artery and fed to the point of interest.

Fluoroscopy enables monitoring of the patient's vascular system and is used to pilot the guide catheter and guide wire to the correct location. Contrast medium is injected, and throughout the dye injection procedure, x-ray images and/or fluoroscopic images (or moving x-rays) are taken to visualise the vascular morphology.

A drawback to the use of contrast medium in angiography is that patients with kidney disease or injury may suffer further kidney damage from the contrast mediums used for angiography. Patients who have blood clotting problems, have a known allergy to contrast mediums, or are allergic to iodine, a component of some contrast mediums, may also not be suitable candidates for an angiography procedure.

While angiography is quite effective for locating large plaque in arteries, this procedure is unable to evaluate whether the plaque is inflamed and/or unstable.

### Summary of the Invention

A vascular catheter apparatus for temperature measurement of vascular tissue, comprising a flexible body, at least two thermal sensors mounted on resiliently biased projections depended from the body, and a carrier for transmitting temperature data at the vascular wall from the sensors to a remote device, is disclosed.

Importantly, it has been reported that unstable and inflamed plaque can cause the temperature of the artery wall to elevate up to 2.5°C proximate the inflamed plaque. With the present disclosure, the vascular catheter apparatus, hereinafter referred to as a thermography catheter, is inserted into the artery to detect the temperature at the vascular wall. The temperature information is subsequently transferred via the carrier to a remote device where the wall temperature can be detected and recorded. Therefore, the present system is able to locate inflamed plaque by monitoring the vascular wall for elevated temperatures. This may be achieved by measuring temperature relative to normal segments of a vessel or absolute temperature values.

Generally, the thermography catheter comprises a plurality of co-axial lumen. Preferably, the thermography catheter comprises a central lumen adapted to be mounted on a standard angioplasty guide wire suitable for vascular intervention. The apparatus is preferably based on the rapid-exchange or the monorail system, although over-the-wire techniques are also envisaged. Preferably, outside the central lumen is located an intermediate lumen. Preferably, outside the intermediate lumen is mounted an external lumen, hereinafter referred to as a sheath. Preferably, at the distal tip of the apparatus is a guide member. Other lumen may be present and all the lumen may house components within themselves or between adjacent lumen.

The projections are preferably mounted on the central or intermediate lumen but may be attached to any lumen inside the sheath.

The central lumen may be formed from the standard catheter lumen materials, for example, nylon, PTFE, polyurethane, polycarbonate and silicones and mixtures thereof.

The intermediate lumen and the sheath are generally constructed from, but individually selected from, the standard catheter lumen materials discussed above.

The sheath is adapted to fit over the adjacent lumen housed inside the sheath and should be able to move relative to the adjacent lumen under the control of a remote device.

Preferably, the central and intermediate lumen are bound to one another and are not moveable relative to one another.

Preferably, the guide member is located at the extreme distal tip and is permanently mounted on the central lumen. Preferably, the guide member is formed from a material which minimises the possibility of damaging the vascular wall. For example, an elastic material is usually used to form the guide member. In particular, preferred materials for the guide member include nylon, PTFE, polyurethane, polycarbonate and silicones. The guide member is usually tapered towards the extreme distal tip and forms a general bullet or pear shape. This enables easy manipulation of the catheter within the vascular tissue and minimises the possibility of potential damage to vascular tissue. The distal part, typically the last 20cm or so, needs to be of sufficient flexibility for the thermography catheter to pass arterial angulations of at least 90° and up to 180° in vessels that may be as small as 2mm, with a curvature radius that may be as low as 4mm.

Preferably, the flexible body of the thermography catheter has a longitudinal axis and at least part of the projections are extensible radially from the longitudinal axis of the body. Generally, the projections have an elongate shape, preferably having dimensions in the range of 2 mm to 15 mm, more preferably 3 to 7 mm in length. The projections preferably have a caliper of 0.3 mm to 5 mm, more preferably 0.5 mm to 3 mm.

A first end of the projection is preferably attached to the body, preferably the intermediate and/or the central lumen, while a second end comprises one or more sensors. The second end is preferably free, ie, not attached to any of the lumen, and is adapted to be radially movable away from the central lumen.

Alternatively, the projection may be attached to a lumen at more than one position, for example at each end of the projection. Such a projection construction forms a loop. In such a case, the sensor is preferably located at the apex of the loop.

Two or more sensors, preferably 2 to 10 sensors, more preferably 2 to 6 sensors may be utilised in the present invention. Preferably, each sensor is mounted on a separate projection. In a particularly preferred example, four projections, each having a single sensor mounted thereon, are provided.

The sensors are preferably located on an outer face of the projection, relative the central lumen, ie., facing the vascular tissue in use. Each sensor should preferably be located toward, or at the distal tip of the projection.

The projections need not be mounted in substantially the same circumferential plane of the thermography catheter body, but this configuration is preferred.

The projections preferably comprise a super elasticmaterial. Superelasticity refers to the ability of certain metals to undergo large elastic deformation. Such compounds favorably exhibit features such as biocompatibility, kink resistance, constancy of stress, physiological compatibility, shape-memory deployment, dynamic interference, and fatigue resistance.

A large number of super-elastic materials may be utilised, however, Ni-Ti ternary alloys are preferred, particularly binary Ni-Ti with between 50.6 and 51.0 atomic percent nickel. While many metals exhibit superelastic effects, Ni-Ti-based alloys appear to be best suited for deployment in the human body due to them being chemically and biologically compatible.

Preferably, the projection, when not restrained will adopt a deployed configuration in which a free end of the projection is extended away from the central lumen. In this deployed configuration, the projection is resiliently biased against the vascular wall in use, thus initiating contact between the sensor and said wall. This achieves an adequate thermal contact with the vascular wall, without substantially compromising blood flow.

In an alternative example, the projections may be mounted to achieve a similar resiliently biased effect. For example, one method of achieving this would be to mount the projections on a spring, preferably a micro-spring, such that when unrestrained, the projection is extended against the vascular wall as discussed above.

The sensors may be any form of temperature sensor and are preferably selected from thermistors, thermocouples, infra red sensors and the like. Preferably, the sensors are thermistors. These are preferably metal alloys having low electrical impedance. Such thermistors prove extremely reliable regarding the relation between the temperature changes and resistance changes.

Generally, the sensors may be attached to the lumen by any means. Each sensor is preferably attached to the end of each projection permanently. For example, each projection may be attached to the lumen by glue, soldering, welding or may be formed integrally with the lumen.

Each sensor is connected to a carrier capable of transferring the information received from the vascular wall. The carrier preferably has a low impedance. The carrier is in electrical connection with the proximal end of the device. The carrier is preferably selected from nickel and copper wire.

Preferably, the thermography catheter comprises a radiopaque marker which aids in the location of the device by fluoroscopy during interventional surgery. More preferably, at least one sensor includes a marker so that it is discernible via fluoroscopy. Most preferably, individual sensors include different marker types, so that using fluoroscopy, the individual sensors can be identified and their spatial orientation and relative location to a desired part of the vessel wall thus clearly defined.

The distal tip may additionally comprise an ultrasound probe system that can give images of the arterial wall. This may be achieved by the incorporation to the distal catheter tip of a phased array of high-frequency ultrasonic crystals or a mechanical sector ultrasound element. In this way, intravascular ultrasound (IVUS) images may be captured simultaneously with the temperature data. This is extremely useful for morphological data acquisition, correctly recognizing the area of interest and for accurate catheter positioning.

The proximal section of the thermography catheter incorporates a connector for coupling the temperature data signals to a remote device such as a personal computer. Preferably, the connector comprises n+1 female plugs to assure proper transmittance of the electrical voltage signal transmitted from the sensors, where n is the number of sensors. These signals are transmitted along the wires from the sensors. The wires are preferably housed within the sheath and are preferably electrically isolated from the patient. Preferably, the wires are housed between the central lumen and the intermediate lumen, within the outer sheath. The n+1 female plugs are connected to n sensor wires and 1 common ground.

Furthermore, a pull-back device for manipulating a multiple lumen catheter, comprising a first lumen mount for holding a first lumen of the catheter, and a second lumen mount for holding a second lumen of the catheter, and a drive mechanism, wherein each of the first and second lumen mounts is selectively connectable to the drive mechanism for both independent and relative movement with respect to the other lumen mount to control the configuration of the catheter, is disclosed.

Preferably, the pull-back device is adapted for use with the vascular catheter apparatus according to the first aspect of the present invention.

The pull-back device enables a guide catheter and the thermography catheter to be stabily mounted. In particular, the pull-back device enables relative movement between the guide catheter and the thermography catheter but, in use, allows the thermography catheter to move relative to the patient and restrains movement of the guide catheter relative to the patient. The pull-back device additionally allows a controlled retraction and positional retention of the associated sheath, thus ensuring atraumatic expansion of the projections on the thermography catheter.

Preferably, the lumen mount of the pull-back device comprise a mount for the guide catheter, a mount for the sheath and a mount for the combined inner and intermediate lumen. Hereinafter, the guiding catheter mount is referred to as mount A, the sheath mount as mount B, and the inner and intermediate lumen mount as mount C.

Mount A preferably has a fixed position during pull-back but preferably should be adjustable. Mount B and C are preferably moveable relative to one another and to mount A. Mount B and C are preferably motor driven, most preferably stepper motor driven. While mount B and C are moveable, they are preferably adapted to enable selective locking in place relative to one another and/or to mount A. Mount B and C are preferably mounted on the drive mechanism. The drive mechanism enables the catheter to be driven towards or away from the patient via movement of mounts B and/or C.

The interlocking of mount B and C ensures that the sheath does not move relative to the lumens housed inside the sheath, thereby ensuring the projections remain in the deployed configuration and engaged with the vascular tissue in the area of interest.

The locking mechanism on the pull-back device includes a restraining mechanism, preferably a stopper rod. This is provided with means for engaging projections within mounts B and/or C. A similar set of projections within the same mounts are used to selectively connect the mounts to the drive rod. These projections may be actuated by a user who can selectively control which of the mounts is locked and which are driven, and the interaction between the mounts.

The drive mechanism is preferably driven by a stepper motor, and preferably gearing is provided along with control and monitoring means.

It is particularly important that substantial occlusion of the vascular tissue is prevented. This is achieved by the present invention as the apparatus in a deployed configuration does not substantially increase its radial cross sectional area beyond the radial cross sectional area of the apparatus in a retracted configuration.

Preferably, the ratio of the area of the cross-sectional profiles of the apparatus in the deployed to retracted configurations is in the range 4:1-1:1, preferably 3:1-1.25:1, more preferably 2.5:1-2:1, most preferably 1.75:1-1.25:1.

The vascular catheter apparatus of the present disclosure, subsequent to the identification and measurement of vascular tissue, in particular, atherosclerotic plaque, may be used to treat an area identified as being at risk of rupture of said plaque. Treatment may be effected by reinserting the catheter to a predetermined area of the vascular tissue. This reinsertion may be achieved in a controlled manner as the prior temperature measurement scan with the device may be used to produce a temperature map of the vascular tissue. This information may be stored in the remote device and can be used to relocate the area of risk. This procedure requires less contrast media to be infused into the patient than would normally be required in similar vascular interventional procedures as the position of the thermography catheter is known due to the data stored in the remote device. The pull-back device may then, under the control of a user, be used to drive the catheter back to, for example, the starting point of the temperature measurement or any point along the path of the temperature data acquisition, for further temperature measurements or alternative treatments of the vascular tissue.

For example, the catheter apparatus can then be used to treat the area by any of the usual therapeutic procedures, including localised delivery of a therapeutic agent, delivery of a stent, brachy therapy, ablation of selected tissue etc. Thus the thermography catheter may additionally comprise angioplasty balloons or sleeves.

According to a first aspect of the present invention, a computer program product comprises computer executable instructions for manipulating image data and temperature data to generate an output in which the temperature data is mapped onto a corresponding position on an image where that temperature data was detected to provide an integrated graphical image output, wherein the temperature data is thermography data that represents surface temperature at a vascular wall, and the image data is representative of the vascular wall morphology.

Furthermore, a method of obtaining temperature data at a vascular wall comprising the steps of withdrawing a thermography catheter that senses vascular wall temperature over a predetermined length of the vascular tissue and processing the temperature data with reference to image data representative of the vascular wall morphology to provide an integrated graphical image output in which the temperature data is mapped onto a corresponding position on the image where that temperature data was detected, is disclosed.

According to a second aspect of the present invention, a computer implemented method of manipulating image data and temperarture data and generating an output, comprising the steps of mapping temperature data onto a corresponding position on an image where the temperature data was detected and generating an integrated graphical image output, wherein the temperature data is thermography data that represents surface temperature at a vascular wall, and the image data is representative of the vascular wall morphology.

Preferably, the image data is one of angiogram image data and intravascular ultrasound image data of the same vascular wall.

Preferably, the thermography data is captured using a vascular catheter apparatus in accordance with the first aspect of the present invention.

Preferably, the integrated graphics image output is a two-dimensional representation of a target vessel morphology with a temperature profile of the target vessel wall overlaid.

Alternatively, the integrated graphics image output may be a three-dimensional representation of the target vessel morphology with a temperature profile of the target vessel wall overlaid.

### Brief Description of the Drawings

Examples of the present invention will now be described in detail with reference to the accompanying drawings, in which:
Figure 1 shows a schematic diagram of a system for conducting vascular catheterisation of a patient;
Figure 2 shows a side view of the distal tip of the device in a temperature sensing (deployed) configuration;
Figure 3 shows a side view of the distal tip of the device in a non-temperature sensing (retracted) configuration;
Figure 4 shows the pull-back device in side view;
Figure 5 shows the pull-back device in plan view;
Figure 6 is a flow diagram illustrating the steps involved with conducting intravascular catheterisation of a patient and the associated data capture and image processing
Figure 7 shows an angiogram frame overlaid with a temperature profile; and
Figure 8 shows an IVUS frame overlaid with a temperature profile.

### Detailed Description

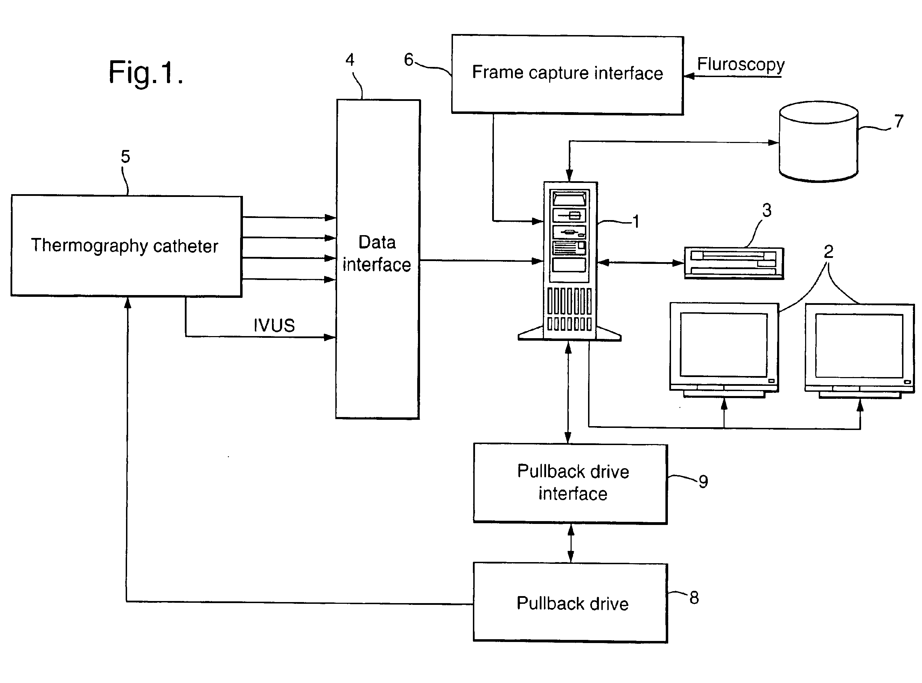

Figure 1 is a schematic diagram of a system for conducting vascular catheterisation of a patient.

The system includes a personal computer (PC) 1 that presents a general user interface (GUI) via a number of monitors 2. The user interface system is based on a Microsoft Windows^{™} platform. Multiple windows may be used to acquire/project data from/to the user. Although not shown, the PC can accept user inputs via a keyboard and mouse, or other pointing device, in the usual manner. The PC includes a number of data stores 7, which may be external, and a CD ROM reader/writer device 3.

The PC is coupled via a data interface 4 to a thermography catheter 5, details of which will be described below. In this example, the thermography catheter 5 transmits four channels (one for each sensor) which are received by the data interface 4. An analogue temperature data signal on each channel is converted to a digital signal using an A/D converter within the data interface 4 at a user configured sampling rate of up to 2.5 KHz. Typically, the sampling rate would be set at around 25 to 50 Hz to reduce the quantity of data acquired.

The data interface 4 includes a multiplexer (not shown) that combines the four digital channels into a single time division multiplexed (TDM) signal. This TDM signal is coupled to the PC over a PCI bus. The data from each channel is written into an area of memory within the data store 7 reserved for that channel where it can subsequently be retrieved for data processing along with the corresponding time sequenced data from other channels and image data from other sources.

The temperature data from the thermography catheter 5 is introduced to the system software running on the PC using function calls. Temperature data are input to the software as the actual voltage at the A/D hardware inputs, and therefore they have to be converted to temperature. A sensor data convert function handles this process.

The system is designed to be used in conjunction with a fluoroscopy x-ray apparatus and therefore includes a video frame capture interface 6 that couples fluoroscopy video data inputs to the PC via a PCI bus. Similarly, it can be used in conjunction with intravascular ultra-sound (IVUS) image data fed from the thermography catheter 5 (when provided with the appropriate hardware). The system software allocates sufficient memory area to the systems memory for this data, taking into account the current system configuration, for example sampling rate, recording time, and video frame size. A memory handle hDib is used to map video data directly through the PCI bus from the video frame capture interface 6 to this allocated area in memory. hDib memory is divided into i equal chunks, each of a size equal to the frame capture interface frame-buffer. Optionally, hDib [i] data can also be mapped to a memory area of a screen-video buffer, giving capability of live preview during recording. Each time the software records an x group of four (or more) temperature measurements, it prompts for a frame capture at hDib [x]. A user configuration file determines the ratio between temperature data:fluoroscopy video frame capture.

Whilst in normal circumstances the thermography catheter 5 is inserted manually, it is intended that when performing vascular measurements the thermography catheter 5 is pulled back relative to a predetermined start position using an electro-mechanical pull-back drive 8 coupled to the body of the catheter. The pull-back drive 8 is controlled by the PC via a pull-back drive interface 9. The system software accesses user-defined configuration files to get the necessary information about controlling the systems automatic pull-back interface 9. Data sampling rate, recording duration and pre-selected retraction rate are taken into consideration for adjusting the pull-back speed. The software routines control a D/A converter (not shown) that feeds the input of the pull-back interface 9 with an appropriate control voltage. The controlled pull-back process will be described in more detail below.

Temperature data plotting may be both on-line and/or off-line. In an on-line mode, the monitor presents a temperature/time-distance graph, where temperature is continuously plotted as connected dots. In an off-line mode, temperature data can be loaded from the data store 7 (or other media) and plotted on the screen graph. The user can scroll to different time/temperature locations, while several automated functions may be provided, for example auto min-max marking, colour coding of temperature on a bullseye graph, colour thermal maps, and 3D temperature coding on a cylinder model. In the latter case, an artificial colour 3D cylinder that represents the vessel is divided into splines equal to the temperature channels. The channel temperature is coded on each spline with colours varying from dark-blue (minimum temperature) to flashing-red (maximum temperature). The user can rotate the cylinder as he wishes in a virtual 3D world. The focus is set to the specific time/distance that corresponds to the mouse position on the screen temperature/time graph. 3D position control is performed using multi cubic-bezier lines, where the curvation control points change in relation to the cylinders position in the virtual world. A separate window shows numeric details for the particular time/distance position. Video frame data from simultaneous fluoroscopy/IVUS are plotted as image frames in a separate window. By moving to a specific time/temperature position, the corresponding video frame is automatically projected. In this way, temperature and video frames are accurately synchronised.

The system software is designed to provide basic and advanced image processing functions for the captured fluoroscopy/IVUS video frames, such as filtering and on-screen measurement functions. The user can filter the captured frame to discard unwanted information while focusing on the desired one. There are several auto-filter options as well as manual adjustment of the image curve. In addition, the user can calibrate the system and proceed in performing on-screen measurements of both distances and/or areas. Automatic routines perform quantification of the measurements giving significant information on lesion characteristics. The temperature can also be colour coded on the fluoroscopy frame, providing unique information about the correlation between temperature and morphology.

By using temperature data and video frame data, the system software uses advanced algorithms based on interpolation and fractal theory to plot a 3D reconstruction of the vessel under measurement with colour coding of temperature. The user can freely move the virtual camera inside the reconstructed vessel in 360°, and/or fly-through the vessel. 2D reconstructions are also provided. Temperature data can be processed on the basis of mean temperature, or on a channel-by-channel basis.

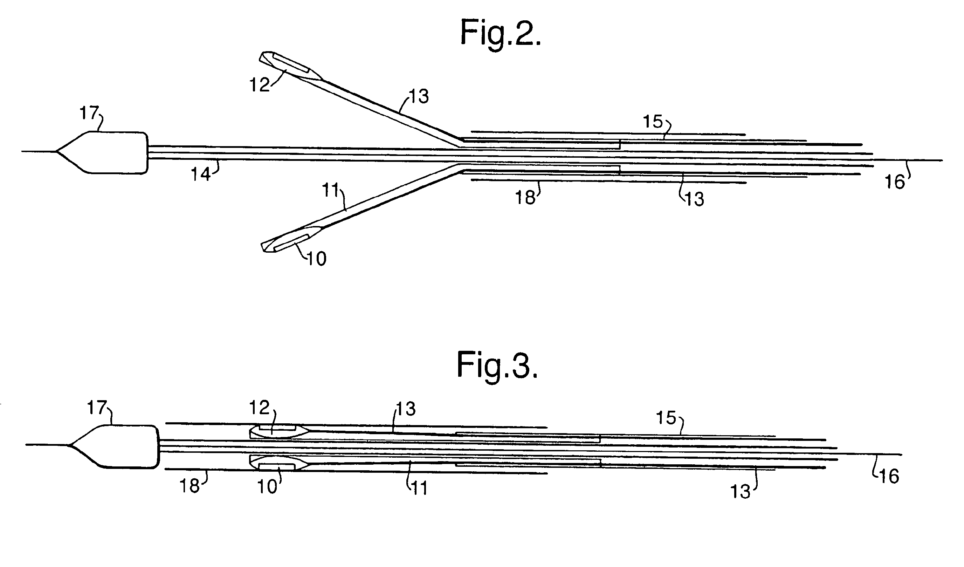

Figure 2 shows one example of the distal tip of a thermography catheter incorporating sensors 10 mounted circumferentially about a central lumen 14. In this example, four sensors 10 are mounted on resiliently biased projections 11 circumferentially about the central lumen at 90° intervals, although only two sensors are shown here for the sake of clarity.

The sensors 10 are NTC thermistors. Such thermistors prove extremely reliable regarding the relation between the temperature changes and resistance changes. An NTC thermistor having a 30 KΩ impedance at 25°C typically maintains linearity between 35°C and 45°C, at a resolution of 0.01°C - 0.1°C.

The construction of the thermistors 10 are that of two rectangular plates with a metal alloy oxide in the centre. The thermistor has dimensions in the range of 0.25mm - 5mm, and a caliper less than 1mm.

Each thermistor 10 is permanently attached to the end of each projection 11 by bonding with an thermally conducting epoxy glue 12. Each thermistor 10 is permanently connected to an insulated wire 13, preferably an insulated bifilar wire. The wire 13 has a low impedence and is constructed from nickel and/or copper. This wire provides an electrical connection with the proximal end of the device (not shown).

The projections 11 are mounted on the central lumen 14 and sandwiched between the central lumen 14 and an intermediate lumen 15. The point at which the projections 11 meet the central/intermediate lumen terminus is sealed. This means that the components located between the central and intermediate lumen are electrically isolated from the patient except through the projections. This also means that no air or debris which may find its way into the space between the lumen can be transmitted to the patient.

As shown in Figure 2, the thermography catheter is mounted on an angioplasty guide 16 wire which runs through the central lumen 14 and a guide member 17 which defines the tip of the thermography catheter.

In use, the apparatus may be actuated between a non-wall-temperature sensing configuration and a temperature sensing configuration. The non-temperature sensing configuration is hereinafter referred to as the retracted configuration. The temperature sensing configuration is hereinafter referred to as the deployed configuration. An example of the deployed configuration is shown in Figure 2. An example of the retracted configuration is shown in Figure 3.

In the retracted configuration, a sheath 18 encompasses the projections 11 so that they are constrained to lie parallel to the longitudinal axis of the catheter and therefore cannot take up a deployed position. The sheath 18 extends as far as the rear end of the guide member 17 but does not overlap the guide member. This minimises any protrusions from the thermography catheter which could lead to damage of the vascular wall. This is particularly important where a vessel is angulated or there is bifurcation of the vessel. Such features lead to bending of the thermography catheter and would emphasise any protrusions. Hence, in this example the sheath 18 and the guide member 17 present a smooth profile when adjacent to one another in the retracted configuration.

To adopt the deployed configuration, the sheath 18 is withdrawn away from the extreme distal tip i.e., away from the guide member 17, towards the proximal section, to expose the projections 11. When the sheath 18 is withdrawn to the extent shown in Figure 2, the resiliently biased projections 11 take up the deployed configuration. It should be noted that the sheath is controlled from the proximal end of the apparatus and is not shown in its entirety in the Figures.

In the deployed configuration, the sheath 18 is retracted until it is at least level with the mountings for the projections 11 on the intermediate lumen 15 so that it does not impede the movement of the projections.

The projections are made of NiTinol and take on the deployed configuration automatically due to their superelastic properties, ie., they are self-expanding.

It should be noted that each projection 11 is effectively independent and thus may extend to the vascular wall in the deployed configuration but will not exert high levels of force upon the wall.

An excessive force should not be exerted on the vascular wall. This will vary between one type of vascular wall and another. In all cases, the apparatus exerts enough force to enable an adequate thermal contact between the sensors 10 and the vascular wall. More particularly, when the catheter is in the deployed configuration, preferably all of the projections 11 are in contact with the vessel wall at any one point in time.

The projections 11 individually extend a certain angle of expansion(r) away from the longitudinal axis of the catheter. In the deployed configuration, r has a value in the range of 15° - 70°. However, r is not fixed and varies with the diameter of the vascular tissue being measured due to the flexibility of the projections 11.

Different diameter catheters may be used for different diameters of vascular tissue. However, as it is desirable to minimize the diameter of catheters in all interventional vascular treatments, it is desirable to adapt the length of the projections and/or the angle to which the projections may extend away from the central lumen depending on the dimensions of the vascular tissue being measured rather than increasing catheter body dimensions. Thus, the projections for a large blood vessel, for example 8 mm diameter, will generally require a length of projection in the range of 5 mm to 10 mm. Smaller diameter vascular tissue, for example 2.5 mm diameter, will generally require a length of projection in the range of 2 mm to 6 mm. Typically, the ratio of the area of the cross-sectional profiles of the apparatus in the deployed to retracted configurations is up to 4:1.

The thermography catheter includes a valve system (not shown) allowing the central lumen 14 to be flushed in an adequate way, thus minimising the possibility of air bubbles or debris within the lumen. Such a valve is constructed to enable engagement by a 2 mm, 5 mm, or 10 mm, 6° luer syringe. The thermography catheter may be flushed with a suitable fluid such as saline. When flushing the catheter, fluid should exit via the distal tip of the catheter, indicating proper flushing of the central lumen 14.

The proximal section of the thermography catheter (not shown) incorporates a connector for the temperature signal transfer to the data interface 4. The connector contains five female plugs to assure proper transmittance of the electrical voltage signal transmitted from the four thermistors 10. These signals are transmitted along the wires 13 from the four thermistors 10. The wires 13 are housed between the central lumen 14 and the intermediate lumen 15, within the outer sheath 18. The five female plugs are connected to four sensor wires and one common ground. A directional, 5 pin, gold plated, water-resistant connector is used.

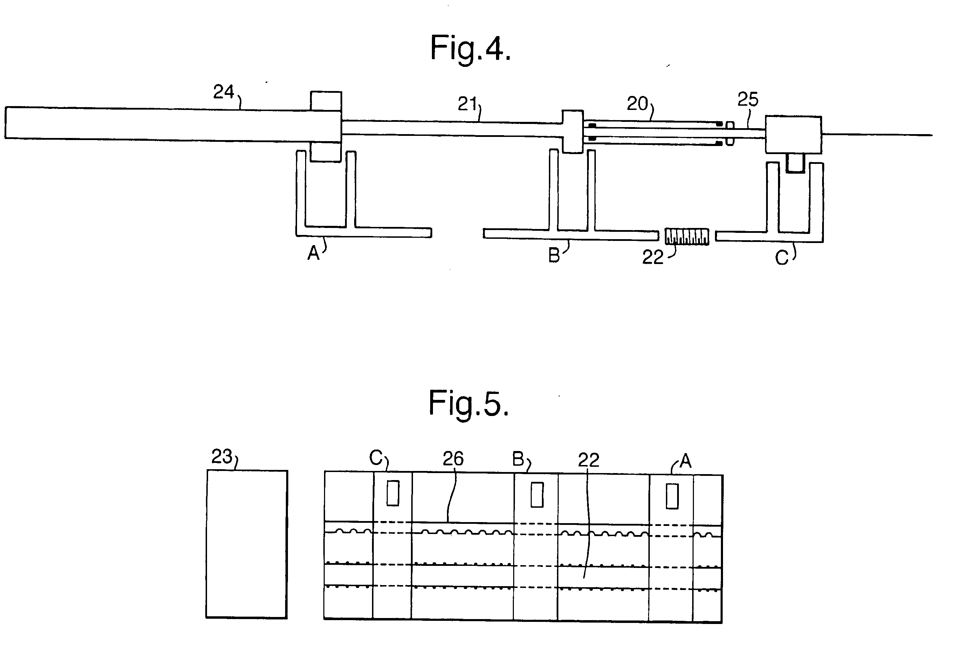

The body of a pull-back device is illustrated in Figure 4 and 5. The proximal section of the thermography catheter described above is constructed to enable remote deployment and retraction of the projections. This is effected via manipulation of the sheath. A two-lumen telescopic construction 20 is used to manipulate the sheath 21 between the retracted and the deployed configuration. One lumen is connected to, or integral with, the outer sheath and can slide over an adjacent lumen which comprises or is connected to one of the lumen housed within the sheath. Rotation of one tube inside the other is prevented by slotting of the lumen. Additionally, scaling markings (not shown), may be provided to avoid over-retraction of the tubes.

The pull-back device includes a drive module 23 which includes a motor, gearing system, typically a speed reducer, control and monitoring means, and engagement gear for a driving rod 22. The drive module is formed separately from the body of the pull-back device so that it may be reused. The body of the pull-back device must be kept sterile and is formed from a material such as polyurethane. This allows the body to be cheaply and easily produced and may be disposable.

The pull-back device comprises a driving rod 22, adapted for engagement with an engagement gear of the drive module 23 and mounts B and C. Mounts B and C are adapted to engage the central/intermediate lumen 25 and the sheath lumen 21 respectively. A Mount A is provided which is adapted to engage the guide catheter 24. When engaged, the mounts B and C may be selectively driven reversibly over a range of travel suitable for inserting the catheter apparatus into a patient and subsequent withdrawal. The driving rod 22 is a worm-screw type which interacts with the engagement gear of the drive module 23, thus providing a smoothly driven apparatus.

The mounts B and C may individually be locked in position relative to one another or may be selectively unlocked in order to allow movement of the lumen 25, sheath 21 and guide catheter 24 relative to one another.

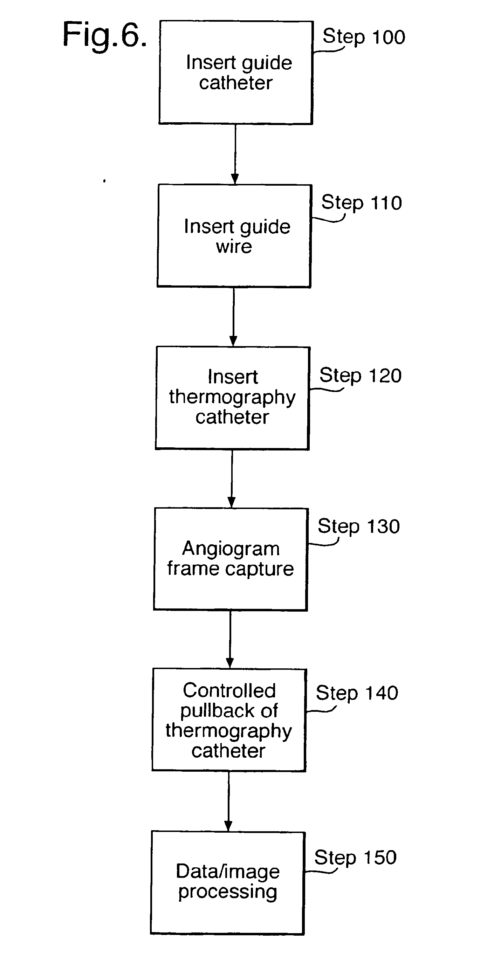

With reference to Figure 6, in use, the sequence of events begins with the insertion of a guiding catheter into the area of general interest (step 100), for example the cardiac region. Where, for example, the coronary arteries are to be examined, the guiding catheter is inserted so that it is adjacent the opening of the coronary arteries (step 110). An angioplasty guide wire is then inserted into the coronary artery, past the point of specific interest. The guiding catheter is usually inserted with the aid of standard fluoroscopic techniques, as is the guiding catheter. Once the guiding catheter and guide wire are in position, the thermography catheter of the present invention is manoeuvered over the guide wire to a position beyond the specific area of interest in the coronary artery (step 120) with the aid of fluoroscopy.

An angiogram is taken (step 130) to assess the position of the thermography catheter in the vascular tissue. This image is saved and the position of the thermography catheter is marked on the image so as to define a starting point for the controlled pull-back step.

The guiding catheter 24 is then locked in position on mount A and both the sheath 21 and the lumen 25 housed in the sheath are locked to mounts B and C respectively, on the pull-back device.

The sheath 21 is then be retracted to allow the projections to adopt the deployed configuration. This is achieved by moving mount B towards mount C. Mount C at this time is locked relative to mount A. Once the sheath 21 is retracted sufficiently to allow expansion of the resiliently biased projections, mounts B and C are locked relative to one another.

Alternatively, the mount B and A are locked in position and C is pulled-back. This is feasible if the sheath 21 is retracted sufficiently (equal or greater than the length of the pull-back distance) to allow the intermediate/central lumen 25 to be retracted in the sheath 21 without the sheath impacting on the projections.

A controlled pull-back of the thermography catheter is then undertaken. This achieved by driving the stepper motor to move both mounts B and C relative to mount A. The interlocking of mount B and C ensures that the sheath 21 does not move relative to the lumen housed inside the sheath, thereby ensuring the projections remain in the deployed configuration and engaged with the vascular tissue in the area of interest.

The locking mechanism includes a stopper rod 26. This is provided with graduations capable of engaging electrically actuated locking pins (not shown) within mounts B and/or C. A similar set of electrically actuated locking pins (not shown) within the same mounts are used to selectively connect the mounts to the drive rod 22. A set of locking pins on any particular mount may not be connected to both the drive rod 22 and the stopper rod 26 simultaneously. Thus, each mount is either in drive or stop mode.

When the mounts B and C are both in drive mode, they move relative to mount A but not to one another. When B or C is in drive mode, one of B or C moves relative to the other and to A. This combination allows pull-back of the thermography catheter relative to the guide catheter 24 whilst controlling the positions of all of these components relative to the patient and to one another.

The thermography catheter may be marked to indicate when the sensors are in a deployed or in a retracted position. This may be achieved by provision of a telescopic tubing 20 with appropriate indicators or by simply marking the extreme deployed or retracted position on the apparatus.

Controlled pull-back of the thermography catheter then takes place (step 140). The pull-back takes place at a constant speed and is controllable by the user. Pull-back typically takes place at speeds of 0.1 to 2 mm in divisions of 0.1 mm or so.

The pull-back takes place over a distance of the vascular tissue being measured. Temperature readings may be taken intermittently or substantially continuously. The data transmitted by the sensors from the vascular wall is captured for data and image processing (step 150) together with a fluoroscopy/IVUS image frame.

As the thermography catheter is withdrawn inside the artery, the projections automatically adjust their angle following the wall's morphology without losing the desired thermal contact. The result is that the thermal contact between the sensors and the wall is continuously maintained, even when the catheter is crossing very irregular plaque formations.

Once the pull-back has been completed, the sheath 21, attached to mount B, is unlocked relative to mount C and extended in order to place the sensors in the retracted configuration. This restores the original smooth profile of the thermography catheter. The sheath mount B can then be locked in place and the thermography catheter reinserted into the same or another blood vessel in order to take another reading. Alternatively, the thermography catheter may be reinserted in order to enable a therapeutic or surgical intervention.

Alternatively, to close the configuration, the mount C is withdrawn so that the projections enter the sheath in the retracted configuration.

As mentioned above, the system software has the capability to capture image-frames that come from standard fluoroscopy or IVUS devices simultaneously with temperature. Spatial data that come from fluoroscopy/IVUS are combined by the software with temperature data. This is done as follows: Before the thermography procedure starts, and while the thermography catheter is still out of the target vessel, the user records the fluoroscopy-tube/bed position and records a video frame during injection of contrast media. The vessel is opacified, and the image is stored and projected on one of the system monitors. The user calibrates the pixel/mm relation by using the guiding catheter as a known reference so that distances in mm can subsequently be estimated accurately on the monitor.

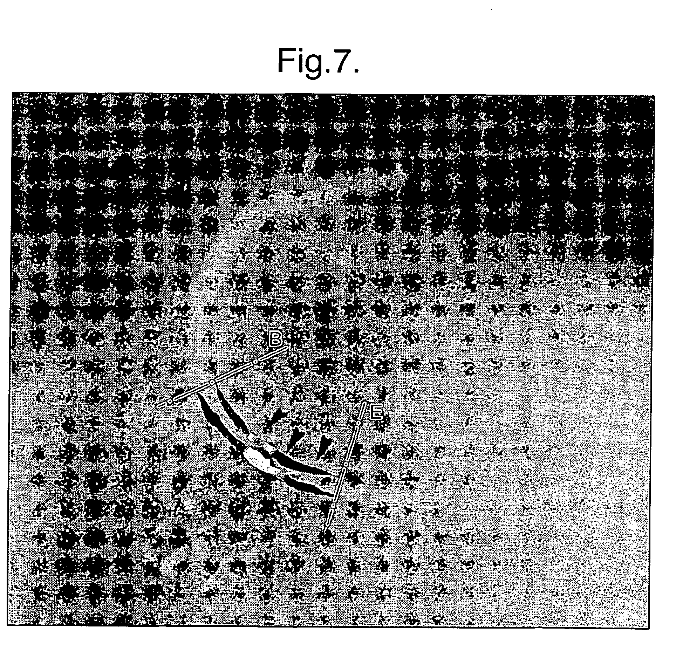

As shown in Figure 7, the user then marks the beginning and ending of the area of interest (points B and E) by clicking on them using the mouse; in return, the software marks these points on the monitor by arrows or lines. The user then positions the thermography catheter in the target vessel by pushing it forward on the guide wire until the fluoroscopic marker on the thermography catheter passes point E over a few mm; while watching the system's monitor, the thermal sensors are then deployed and the user manually pulls the thermography catheter back gently until the fluoroscopic marker overlaps exactly on point E. The software then instructs the automatic pull-back device to pull back the thermography catheter over the length of the BE curve within the vessel.

The software then performs auto-border detection on the BE area of the fluoroscopy video frame using a photoluminescence technique, and temperature is subsequently coded in the atherosclerotic plaque outline as RGB color degradation from dark-blue (0,0,255) corresponding to the minimum detected temperature, to flashing red (255,0,0) corresponding to the maximum detected temperature. This is illustrated in Figure 7. A reference color map may be provided, and by moving the mouse cursor inside the BE area, temperature values may also automatically be provided in a numeric format.

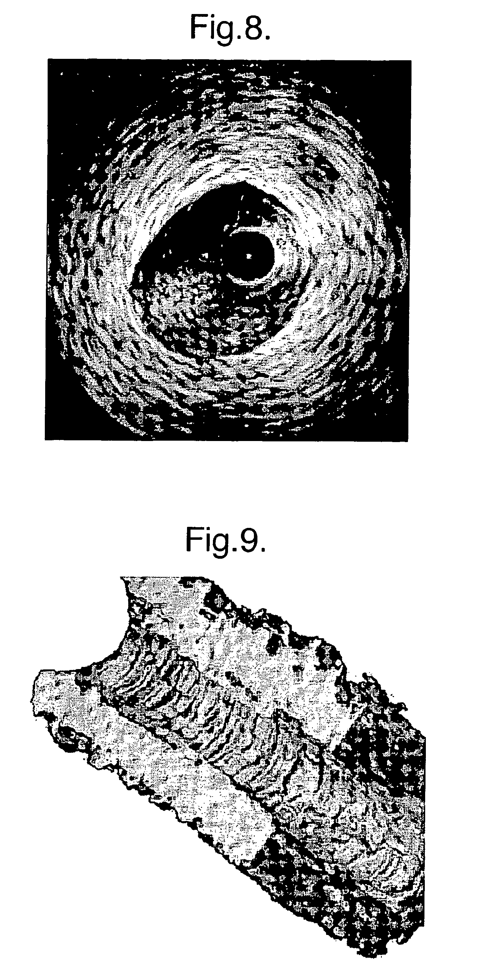

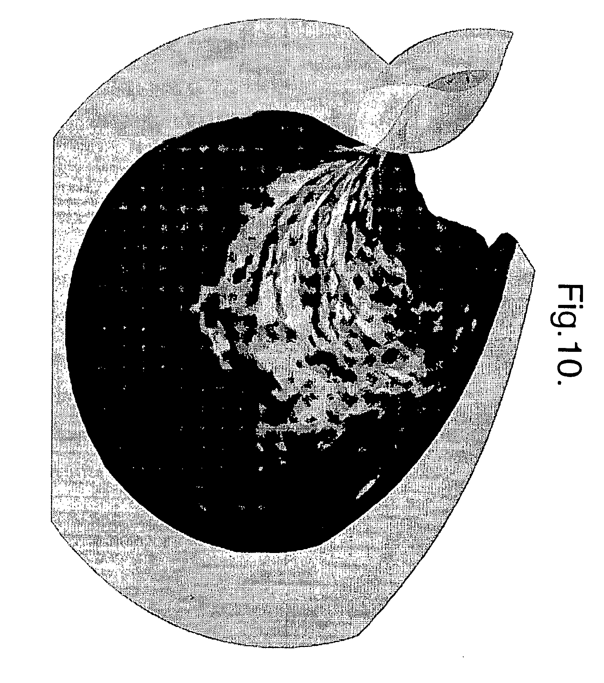

Figure 8 shows the same image processing as applied to a single IVUS image frame for a section of the target vessel. Figure 9 shows a 3D section of a target vessel constructed using a series of IVUS images (without temperature mapping). As shown in figure 10, a number of IVUS images can be processed to provide a 3D representation of the temperature profile/morphology over a length of the target vessel.

## Claims

1. A computer program product comprising computer executable instructions for manipulating image data and temperature data to generate an output in which the temperature data is mapped onto a corresponding position on an image where that temperature data was detected to provide an integrated graphical image output, wherein the temperature data is thermography data that represents surface temperature at a vascular wall, and the image data is representative of the vascular wall morphology.

2. A computer implemented method of manipulating image data and temperature data and generating an output, comprising the steps of mapping temperature data onto a corresponding position on an image where the temperature data was detected and generating an integrated graphical image output,
wherein the temperature data is thermography data that represents surface temperature at a vascular wall, and the image data is representative of the vascular wall morphology.

3. A computer program product or a method according to claim 1 or 2, wherein the image data is one of angiogram image data and intravascular ultrasound image data of the same vascular wall.

4. A computer program product or a method according to claim 1 or 2, wherein the integrated graphics image output is a two-dimensional representation of the target vessel morphology with a temperature profile of the target vessel wall overlaid.

5. A computer program product or a method according to claim 1 or 2, wherein the integrated graphics image output is a three-dimensional representation of the target vessel morphology with a temperature profile of the target vessel wall overlaid.

## Patentansprüche

1. Computerprogrammprodukt mit computerausführbaren Befehlen zum Verarbeiten von Bild- und Temperaturdaten, um eine Ausgabe zu erzeugen, bei der die Temperaturdaten einer entsprechenden Position auf einem Bild zugeordnet werden, an der diese Temperaturdaten erfasst wurden, um eine integrierte graphische Bildausgabe bereitzustellen, wobei die Temperaturdaten Thermographiedaten sind, die die Oberflächentemperatur an einer Gefäßwand repräsentieren, und wobei die Bilddaten kennzeichnend für die Morphologie der Gefäßwand sind.

2. Computerimplementiertes Verfahren zum Verarbeiten von Bild- und Temperaturdaten und zum Erzeugen einer Ausgabe, das die Schritte des Zuordnens von Temperaturdaten zu einer entsprechenden Position auf einem Bild, an der die Temperaturdaten erfasst wurden, und des Erzeugens integrierten graphischen Bildausgabe aufweist,
wobei die Temperaturdaten Thermographiedaten sind, die - die Oberflächentemperatur an einer Gefäßwand repräsentieren, und wobei die Bilddaten kennzeichnend für die Morphologie der Gefäßwand sind.

3. Computerprogrammprodukt oder Verfahren nach Anspruch 1 oder 2, wobei die Bilddaten Angiogramm-Bilddaten oder intravaskuläre Ultraschallbilddaten derselben Gefäßwand sind.

4. Computerprogrammprodukt oder Verfahren nach Anspruch 1 oder 2, wobei die integrierte graphische Bildausgabe eine zweidimensionale Darstellung der Morphologie des Zielgefäßes ist, wobei ein Temperaturprofil der Zielgefäßwand darüber gelegt ist.

5. Computerprogrammprodukt oder Verfahren nach Anspruch 1 oder 2, wobei die integrierte graphische Bildausgabe eine dreidimensionale Darstellung der Morphologie des Zielgefäßes ist, wobei ein Temperaturprofil der Zielgefäßwand darüber gelegt ist.

## Revendications

1. Produit de programme informatique comprenant des instruction exécutables par ordinateur pour manipuler des données d'image et des données de température pour générer un produit de sortie dans lequel les données de température sont mappées sur une position correspondante sur une image sur laquelle ces données de température sont détectées pour donner une sortie d'image graphique intégrée, dans lequel les données de température sont des données thermographiques qui représentent une température de surface au niveau d'une paroi vasculaire et les données d'image sont représentatives de la morphologie de la paroi vasculaire.

2. Procédé implémenté par ordinateur, de manipulation de données d'image et de données de température générant un produit de sortie, comprenant les étapes consistant à mapper des données de température sur une position correspondante sur une image sur laquelle les données de température ont été détectées et à générer une sortie d'image graphique intégrée,
dans lequel les données de température sont des données thermographiques qui représentent une température de surface au niveau d'une paroi vasculaire et les données d'image sont représentatives de la morphologie de la paroi vasculaire.

3. Produit de programme informatique ou procédé selon les revendications 1 ou 2, dans lequel les données d'image sont des données d'angiographie et des données d'images échographiques intravasculaires de la même paroi vasculaire.

4. Produit de programme informatique ou procédé selon les revendications 1 ou 2, dans lequel la sortie d'image graphique intégrée est une représentation bidimensionnelle de la morphologie d'un vaisseau cible avec un profil de température de la paroi du vaisseau cible en cascade.

5. Produit de programme informatique ou procédé selon les revendications 1 ou 2, dans lequel la sortie d'image graphique intégrée est une représentation tridimensionnelle de la morphologie d'un vaisseau cible avec un profil de température de la paroi du vaisseau cible en cascade.
